# EUROPEAN PATENT APPLICATION

(11) **EP 1 527 773 A1**
(43) Date of publication of application: **04.05.2005**
(21) Application number: 05075288.0
(22) Date of filing: 04.12.2002
(51) Int. Cl.: A61K 9/22, A61K 9/46, A61K 31/00

(54) **Pharmaceutical composition comprising sumatriptan**

(30) Priority: 05.12.2001 GB 0129117
(62) Divisional of application: 02792887.8
(71) Applicant: GLAXO GROUP LIMITED, Greenford, Middlesex UB6 ONN (GB)
(72) Inventor: Baker, Robert William, Ware Hertfordshire SG12 0DP (GB); Dow, Alan David, Ware Hertfordshire SG12 0DP (GB); Summers, Simon John, Ware Hertfordshire SG12 0DP (GB); Westrup, Julian, Third Avenue Harlow Essex CM9 5AW (GB)
(74) Representative: Reed, Michael Antony

(57) **Abstract**

The present invention relates to a pharmaceutical composition for oral administration capable of rapid disintegration and dispersion within the gastrointestinal tract comprising sumatriptan or a pharmaceutically acceptable salt or solvate thereof as active ingredient, in particular a composition in solid-dosage form that is intended to be swallowed, and methods of treatment of cephalic pain, especially migraine, using such composition.

## Description

The present invention relates to a pharmaceutical composition for oral administration capable of rapid disintegration and dispersion within the gastrointestinal tract comprising a 5HT₁ receptor agonist as active ingredient, in particular a composition in solid-dosage form that is intended to be swallowed.

5HT₁ receptor agonists are useful in the treatment of conditions associated with cephalic pain such as migraine, cluster headache, chronic paroxysmal hemicrania, headache associated with vascular disorders, headache associated with substances or their withdrawal, rebound headache and tension headache. 5HT₁ receptor agonists are well known in the art and the term is to be broadly understood to include 5HT₁ receptor agonists of all types including, but not limited to, 5HT₁-like receptor agonists, 5HT_{1B} receptor agonists, 5HT_{1D} receptor agonists and 5HT_{1F} receptor agonists. Particular reference is made to the compounds sumatriptan (described for example in GB Patent No. 2162522, incorporated herein by reference), naratriptan, rizatriptan, zolmitriptan, frovatriptan, eletriptan, almotriptan, avitriptan, donitriptan, alniditan, ALX-0646, LY334370, U1092291, IS159 and PNY142633. Especial reference is made to the compound sumatriptan.

An extensive worldwide clinical trials program has demonstrated the efficacy and tolerability of sumatriptan (marketed in subcutaneous, oral, intranasal and rectal formulations) as an acute treatment for migraine.

With sumatriptan subcutaneous injection (6mg), a statistically significant difference from placebo in headache relief is seen as early as 10 minutes after dosing; headache relief at 2 hours post-injection has been reported by 70-82% of patients using this dosage form.

With sumatriptan nasal spray (20mg), a statistically significant difference from placebo in headache relief is seen as early as 15 minutes after dosing; headache relief at 2 hours post-dose has been reported by 55-64% of patients using this dosage form.

With the currently marketed sumatriptan tablets (50mg and 100mg), a statistically significant difference from placebo in headache relief is seen at 30 minutes after dosing; headache relief at 4 hours post-dose has been reported by about 65-78% of patients using this dosage form.

WO92/15295 (Glaxo Group Limited), incorporated herein by reference, discloses known solid-dosage form pharmaceutical compositions, and in particular film-coated tablets, useful in the treatment of conditions associated with cephalic pain.

The pharmacokinetics of sumatriptan are well known. Following oral dosing of sumatriptan solution to healthy volunteers, drug absorption is rapid, with maximum plasma concentrations achieved in approximately an hour. Currently marketed tablet formulations (marketed as Imigran® and Imitrex®) reach median maximum plasma concentrations a little later, sometimes up to 2 hours post-dose. Previous pharmacokinetic studies with a variety of oral formulations of sumatriptan, including solutions, demonstrated similar exposure to the drug as measured by the area under concentration-time curve (AUC) for the time period 0-infinity, and similar maximum observed plasma concentrations (Cmax) compared to the currently marketed tablet. Food has no significant effect on the overall bioavailability of sumatriptan administered orally, but delays Tmax (time to maximum observed serum concentrations) by about 0.5 hour; this is typical of most compounds absorbed in the small intestine because food delays gastric emptying. A similar delay is observed when sumatriptan is administered to patients during a migraine attack. It is believed that gastric stasis as a result of a migraine attack is responsible for the change in pharmacokinetic profile. This delay results in lower systemic concentrations during the initial absorption phase. A reduced disintegration (tablet erosion) rate for the currently marketed sumatriptan tablets in the stomach and delayed stomach emptying are likely consequences of gastric stasis. Any delay in the absorption of a migraine treatment is likely to delay onset of pain relief.

In Fuseau E. *et al.,* Clinical Therapeutics., 23(2), 242-51, Feb. 2001 the effect of encapsulation on the absorption of sumatriptan tablets in healthy volunteers and patients during a migraine was studied. It was concluded that encapsulation delayed sumatriptan absorption; the concentrations observed during the first two hours post-dose were not equivalent and were lower when the tablet was encapsulated. Encapsulation was thus associated with a trend toward slower onset of efficacy as measured by relief of headache and nausea and time to meaningful relief.

Oral administration constitutes a preferred route for administration of pharmaceuticals since this route is particularly convenient and acceptable to patients. Unfortunately, as indicated above, currently marketed oral compositions of 5HT₁ receptor agonists may be associated with certain disadvantages in the treatment of conditions associated with cephalic pain. For example, such conditions, particularly migraine, are associated with nausea, vomiting and gastrointestinal dysfunction in the form of reduced gastric motility and delayed gastric emptying which potentially lead to a delay and/or an impairment of the rate of drug absorption.

WO92/11003 (Laboratoires Glaxo), incorporated herein by reference, discloses effervescent pharmaceutical compositions for oral use useful in the treatment of conditions associated with cephalic pain. Such effervescent compositions are intended to be dissolved and/or dispersed in an aqueous medium prior to being taken by the patient. The effervescent compositions disclosed in WO92/11003 result in the drug being more rapidly absorbed into the plasma when compared to conventional tablet formulations.

However, an effervescent tablet formulation requires water to allow you to take the medication, i.e. it has to be dissolved/suspended prior to swallowing. This is not considered commercially attractive from the perspective of patient convenience and, for a patient who suffers from e.g. migraines in everyday circumstances, it can be indiscreet to have to use such a formulation type.

In addition, where the active ingredient has an inherently bitter taste (and especially where such a bitter-tasting active ingredient has a recommended therapeutic dose of 25mg or greater, as is the case for sumatriptan and its pharmaceutically acceptable salts and solvates thereof) a key disadvantage of formulations where an effervescent tablet is first dissolved in water prior to swallowing is that the bitter taste makes the solution unpalatable; taste-masking of such solutions is difficult to accomplish successfully. Taking an unpalatable medicament is especially problematic for patients suffering from cephalic pain, and especially from migraine, due to the increase in nausea that would result, which in turn would lead to an increased likelihood of vomiting.

In addition, traditional effervescent tablets generally need to be prepared in an especially dry environment, i.e. require more manufacturing controls. Traditional effervescent tablets also tend to be particularly friable and in general in commercial manufacture have to be packed quickly into non-permeable packaging to avoid moisture ingress and/or tablet breakup.

Although traditional effervescent tablets can, in theory, be swallowed directly, in practice this is not recommended and in addition would present further disadvantages. Such effervescent tablets tend to be relatively large and thus difficult to swallow; such effervescent tablets are also generally uncoated (and are not easily film-coated by conventional methods, and especially cannot be provided with an aqueous film coat) and, as indicated above, are particularly friable. Thus, where the active ingredient has an inherently bitter taste (and especially where such a bitter-tasting active ingredient has a recommended therapeutic dose of 25mg or greater, as is the case for sumatriptan and its pharmaceutically acceptable salts and solvates thereof) these characteristics are likely to mean a tablet swallowed in this way would be unpalatable. In addition, there would be a disadvantage caused by the extent of effervescence produced in swallowing such a tablet directly, in terms of distending the stomach, increased gas in the oesophagus, and increased nausea. Again, these disadvantages would be especially problematic for a patient suffering from cephalic pain, and in particular migraine, where unpalatability and/or increased gas and/or increased nausea would increase the likelihood of vomiting.

It would be highly advantageous to have a medicament that combined the convenience to the patient of a tablet formulation that may be swallowed whole with the benefit of the rapid absorption properties of an effervescent formulation. Such a formulation would be particularly advantageous in the treatment of conditions associated with gastrointestinal dysfunction such as migraine and conditions where rapid disintegration and dispersion of an orally administered composition is required.

The present invention relates to a fast disintegrating and dispersing solid-dosage form pharmaceutical composition which has an improved pharmacokinetic profile (an increased rate of absorption) relative to currently marketed 5HT₁ receptor agonist solid-dosage formulations (especially the currently marketed sumatriptan solid-dosage tablet formulations); such a composition is expected to provide faster onset of action and/or higher efficacy rates for a patient suffering from cephalic pain, especially migraine. The present invention thus provides a unique solution to the problem of gastric stasis in such a patient.

It has surprisingly been found that the rate of dissolution of a 5HT₁ receptor agonist from an orally administered composition can be enhanced by the use of an effervescent couple in combination with a disintegrant, an insoluble filler and a wicking agent. The enhanced dissolution rate has also been shown to result in improved absorption *in vivo.*

Accordingly, the present invention provides in a first aspect a solid-dosage form pharmaceutical composition for oral administration comprising a compound which acts as a 5HT₁ receptor agonist, or a pharmaceutically acceptable derivative thereof, as active ingredient together with the base component of an effervescent couple, a disintegrant and an insoluble filler, wherein the base component comprises from about 5 to about 50 % by weight, the disintegrant comprises from about 0.5 to about 10 % by weight, and the insoluble filler comprises from about 30 to about 99 % by weight, said insoluble filler including a wicking agent which comprises from about 1 to about 99 % by weight, based on the dry weight of the dosage form, wherein greater than about 70 %, preferably about 80 %, more preferably about 90 % of the active ingredient is dissolved in simulated gastric fluid (SGF) within five minutes in USPII apparatus at the discriminating paddle speed of 10 rpm.

In preferred embodiments of the present invention, the extent of effervescence (volume of gas) produced is considerably less than that produced by traditional effervescent tablets. In particular, as indicated below, the % amount of the base component can be significantly less than 50%, and also the absolute amount of this component in a single unit of the dosage form will typically be much lower than in traditional effervescent tablets in view of the smaller size of the overall unit dosage form (typically a single unit of the dosage form has a target compression weight of between about 100 mg and about 600 mg, preferably between 150 mg and 450 mg).

In the context of the present invention, by "for oral administration" is meant that the pharmaceutical composition is in a solid-dosage form that is intended to be swallowed whole, and is not primarily intended for dissolution or suspension in water prior to administration; however, the composition of the present invention may in certain embodiments meet the requirements of a dispersible tablet in terms of fineness of dispersion and rate of dispersion as defined by the European Pharmacopoeia and/or British Pharmacopoeia. Such dosage forms may take the form of tablets and capsules and may be prepared according to conventional techniques well known in the art of pharmacy for the manufacture of solid-dosage forms. Preferably, the composition of the present invention is in the form of a 'swallow' tablet. For the avoidance of doubt, a 'swallow' tablet is a tablet which is intended to be swallowed whole (generally with a small amount of liquid, e.g. water); it is not one which is primarily intended for dissolution or suspension in water prior to administration (such as, for example, a tablet as described in WO92/11003 and which also contains a substantial quantity of both components of an effervescent couple), and also it is not one which is primarily intended for dissolution 'in the mouth' (in an 'oral melt' form).

By pharmaceutically acceptable derivative is meant any pharmaceutically acceptable salt, solvate, ester or amide, or salt or solvate of such ester or amide of the 5HT₁ receptor agonist or any other compound which upon administration to the recipient is capable of providing (directly or indirectly) the 5HT₁ receptor agonist or an active metabolite or residue thereof.

Suitable pharmaceutically acceptable salts according to the invention include acid addition salts formed with inorganic acids such as hydrochlorides, hydrobromides, phosphates and sulfates and with organic acids, for example tartrates, maleates, fumarates, succinates and sulfonates.

Suitable 5HT₁ receptor agonists for use according to the invention include sumatriptan, naratriptan, zolmitriptan, eletriptan, rizatriptan, frovatriptan, almotriptan, avitriptan, donitriptan, alniditan, ALX-0646, LY334370, U1092291, IS159 and PNY142633. Naratriptan and sumatriptan are preferred, with sumatriptan being particularly preferred. A preferred form of sumatriptan is the succinate salt, particularly the 1:1 succinate. The 5HT₁ receptor agonists may be used alone or in combination with each other.

Suitably, the 5HT₁ receptor agonist when used according to the invention comprises from about 0.001 to about 55 % by weight, preferably about 0.01 to about 45 %, more preferably about 0.1 to about 40 %, especially about 1 to about 35 %, more especially about 20 to about 35 %, based on the dry weight of the dosage form.

The meaning of the terms disintegration and dispersion as used herein will be well understood by the skilled addressee.

In one embodiment of the present invention, the pharmaceutical composition further comprises the acid component of an effervescent couple.

An effervescent couple consists essentially of an acid component and a base component, which components react in the presence of water to form a gas. The acid component may comprise, for example, the 5HT₁ receptor agonist or a pharmaceutically acceptable derivative thereof itself (where it has an acidic character or can provide a component with acidic character in an aqueous environment), or an aliphatic carboxylic acid or a salt thereof, such as citric or tartaric acid and salts thereof. Alternatively, the acid component may be provided by the stomach acid rather than being part of the pharmaceutical composition. Preferably, the acid component is the 5HT₁ receptor agonist or a pharmaceutically acceptable derivative thereof (such as sumatriptan or naratriptan, especially in the form of salts thereof, for example the succinate salt, such as sumatriptan succinate (1:1)). The acid components may be used alone or in combination with each other. Suitably, the acid component comprises the 5HT₁ receptor agonist or a pharmaceutically acceptable derivative thereof, together with an aliphatic carboxylic acid or a salt thereof, such as citric or tartaric acid and salts thereof. Suitably, the acid component (including the 5HT₁ receptor agonist or pharmaceutically acceptable derivative thereof when it is functioning as an acid component) comprises up to about 55 % by weight, preferably from about 5 to about 50 %, more preferably about 10 to about 45 %, especially about 15 to about 40 %, more especially about 20 to about 35 %, based on the dry weight of the dosage form. The base component may comprise, for example, an alkali metal or alkaline earth metal carbonate or bicarbonate, such as sodium bicarbonate, potassium bicarbonate, magnesium carbonate or calcium carbonate. The base component is preferably sodium bicarbonate. The base components may be used alone or in combination with each other. Suitably, the base component comprises from about 5 to about 50 % by weight, preferably about 7 to about 20 %, more preferably about 8 to about 15 %, especially about 9 to about 12 %, based on the dry weight of the dosage form. Acids may be monoprotic or polyprotic; similarly, bases may be monobasic or polybasic. Calculated in terms of acid/base normalities (N), the ratio of acid component to base component may conveniently be within the range of from about 1:10 to about 10:1, preferably from about 1:5 to about 5:1, more preferably about 1:3 to about 3:1, most preferably about 1:2 to about 2:1.

Disintegrants, when used in the compositions of the present invention, swell when they come into contact with water. Suitable disintegrants will be well known to the person skilled in the art and a non-limiting list of examples includes croscarmellose sodium, sodium starch glycollate, cross-linked polyvinylpyrrolidone, povidone, starch (e.g. maize starch, pregelatinised starch), low substituted hydroxypropylcellulose, alginic acid, sodium alginate, tribasic calcium phosphate, calcium sulfate, calcium carboxymethylcellulose, microcrystalline cellulose, powdered cellulose, colloidal silicon dioxide, docusate sodium, guar gum, hydroxypropyl cellulose, magnesium aluminium silicate, methylcellulose, polacrilin potassium and polyvinyl pyrrolidone. Croscarmellose sodium is preferred. The disintegrants may be used alone or in combination with each other. Suitably, the disintegrant comprises from about 0.5 to about 10 % by weight, preferably about 2 to about 8 %, more preferably about 3 to about 7 %, especially about 4 to about 6 %, more especially about 5 %, based on the dry weight of the dosage form.

Insoluble fillers are inert substances that provide bulk and stability when used in the compositions of the present invention. Some insoluble fillers may also act as wicking agents. Wicking agents, when used in the compositions of the present invention, have a porous nature and draw water into and throughout the solid dosage form. Suitable wicking agents will be well known to the person skilled in the art and a non-limiting list of examples includes microcrystalline cellulose (available as, for example, Avicel™), croscarmellose sodium, crospovidone, starch, calcium carboxymethylcellulose, silicified microcrystalline cellulose, magnesium oxide and tragacanth. Microcrystalline cellulose is preferred. The wicking agents may be used alone or in combination with each other. Suitably, the wicking agent comprises from about 1 to about 99 % by weight, preferably about 1 to about 80 %, more preferably about 5 to about 65 %, especially about 12 to about 55 %, more especially about 18 to about 50 %, based on the dry weight of the dosage form. Other suitable insoluble fillers include dibasic calcium phosphate dihydrate, anhydrous dibasic calcium phosphate (available as, for example, Emcompress™), tribasic calcium phosphate, calcium carbonate, magnesium carbonate, calcium sulfate, cellulose acetate, powdered cellulose, kaolin, polymethacrylates and talc. Anhydrous dibasic calcium phosphate is preferred. The insoluble fillers may be used alone or in combination with each other. Suitably, the insoluble filler, including the wicking agent, comprises from about 30 to about 99 % by weight, preferably about 35 to about 80 %, more preferably about 40 to about 70 %, especially about 45 to 65 %, based on the dry weight of the dosage form.

Thus, in an embodiment of the present invention, there is provided a solid-dosage form pharmaceutical composition as hereinbefore described wherein the 5HT₁ receptor agonist or a pharmaceutically acceptable derivative thereof comprises from about 0.001 to about 55 % by weight, preferably about 0.01 to about 45 %, more preferably about 0.1 to 40 %, especially about 1 to about 35 %, more especially about 20 to about 35 %, the base component of the effervescent couple comprises from about 5 to about 50 % by weight, preferably about 7 to about 20 %, more preferably about 8 to about 15 %, especially about 9 to about 12 %, the disintegrant comprises from about 0.5 to about 10 % by weight, preferably about 2 to about 8 %, more preferably about 3 to about 7 %, especially about 4 to about 6 %, more especially about 5 %, the insoluble filler, including the wicking agent, comprises from about 35 to about 80 % by weight, preferably about 40 to about 70 %, more preferably about 45 to about 65 %, and the wicking agent comprises from about 1 to about 80 % by weight, preferably about 5 to about 65 %, more preferably about 12 to about 55 %, especially about 18 to about 50 %, based on the dry weight of the dosage form.

In a further embodiment of the present invention, there is provided a pharmaceutical composition as hereinbefore described wherein the 5HT₁ receptor agonist or a pharmaceutically acceptable derivative thereof comprises sumatriptan or naratriptan or a pharmaceutically acceptable derivative thereof, preferably sumatriptan or a pharmaceutically acceptable derivative thereof, more preferably in the form of its succinate (1:1) salt, the base component of the effervescent couple comprises sodium bicarbonate, the disintegrant comprises croscarmellose sodium and the insoluble filler comprises microcrystalline cellulose.

In a further embodiment of the present invention, there is provided a solid-dosage form pharmaceutical composition as hereinbefore described wherein the 5HT₁ receptor agonist or a pharmaceutically acceptable derivative thereof comprises sumatriptan or naratriptan or a pharmaceutically acceptable derivative thereof, preferably sumatriptan or a pharmaceutically acceptable derivative thereof, more preferably in the form of its succinate (1:1) salt, the base component of the effervescent couple comprises sodium bicarbonate, the disintegrant comprises croscarmellose sodium and the insoluble filler comprises dibasic calcium phosphate, preferably anhydrous dibasic calcium phosphate.

In a further embodiment of the present invention, there is provided a solid-dosage form pharmaceutical composition as hereinbefore described wherein the 5HT₁ receptor agonist or a pharmaceutically acceptable derivative thereof comprises sumatriptan or a pharmaceutically acceptable derivative thereof, preferably in the form of its succinate (1:1) salt, the base component of the effervescent couple comprises sodium bicarbonate, the disintegrant comprises croscarmellose sodium, and the insoluble filler comprises anhydrous dibasic calcium phosphate or microcrystalline cellulose or a mixture thereof.

The inclusion of one or more insoluble fillers in the compositions of the present invention also provides the composition with improved handling properties during manufacture as compared with a conventional tablet formulation (for example, a formulation as disclosed in WO92/15295).

In addition to the ingredients described above, the pharmaceutical composition of the present invention may further comprise pharmaceutically acceptable carriers and excipients such as binding agents (e.g. pregelatinised starch, polyvinylpyrrolidone, hydroxypropylmethylcellulose) and lubricants (e.g. stearic acid, magnesium stearate, talc, sodium benzoate and hydrogenated vegetable oil).

Although pharmaceutical compositions according to the present invention, and in particular tablet compositions, will in general require non-permeable (e.g. foil/foil) packaging for commercial long-term storage, in certain embodiments (and in particular those embodiments where the 5-HT₁ receptor agonist is sumatriptan (1:1) succinate salt and no other and/or no stronger conjugate acid component is present) they are able to withstand storage in an open vessel in 30°C/60% RH environmental conditions for at least 1 month and can generally be manufactured in standard tabletting facilities i.e. with normal controls and no especially onerous low humidity requirements.

Many drug substances have an inherently bitter taste. The unpleasant taste that is sometimes associated with oral administration of a composition comprising a 5HT₁ receptor agonist or a pharmaceutically acceptable derivative thereof may be substantially eliminated by the use of a film coat on the solid core. The solid core comprises the 5HT₁ receptor agonist or a pharmaceutically acceptable derivative thereof. Moreover, when used in the compositions of the present invention, the film coat delays the disintegration of the solid dosage form until it reaches the stomach. A film coat can also aid swallowing, can make the solid dosage form aesthetically more pleasing, and generally makes the solid-dosage form less friable.

Accordingly, in one embodiment, the present invention provides a pharmaceutical composition as hereinbefore described in the form of a tablet which is film-coated.

The film coating may suitably comprise a polymer. Suitable polymers will be well known to the person skilled in the art and a non-limiting list of examples includes cellulose ethers, for example, hydroxypropylmethyl cellulose, hydroxypropyl cellulose or methylcellulose, and copolymers of methacrylic acid and methyl methacrylate. Preferably, the film coating will comprise hydroxypropylmethyl cellulose.

The total film coating solids are generally applied to the solid dosage form, for example the tablet core, in an amount of from about 0.5 to 10 % by weight, preferably about 1 to about 4 %, more preferably about 2 to about 3 %, based on the dry weight of the dosage form. For example, about 8 mg of coat is used for a tablet core weighing about 300 or about 400 mg, and about 4 mg of coat is used for a tablet core weighing about 175 mg.

The film coating may additionally comprise any pharmaceutically acceptable colourants or opacifiers including water soluble dyes, aluminium lakes of water soluble dyes and inorganic pigments such as titanium dioxide and iron oxide.

The film coating may also contain one or more plasticising agents conventionally used in polymeric film coatings, for examples, polyethylene glycol, propylene glycol, dibutyl sebecate, mineral oil, sesame oil, diethyl phthalate and triacetin. Proprietary film coating materials, such as Opaspray and Opadry, obtainable from Colorcon Ltd., UK, may be used.

The taste of oral compositions may also be improved by the use of flavouring and/or sweetening agents. Suitable flavouring agents will be well known to the person skilled in the art and a non-limiting list of examples includes lemon, orange, grapefruit, vanilla, caramel, butterscotch, hazelnut or mint flavouring. Suitable sweetening agents for use in the compositions of the invention will be well known to the person skilled in the art and a non-limiting list of examples includes sucrose, saccharin, cyclamic acid and alkali or alkali earth metal salts thereof, mannitol, acesulfame-K, stevioside, thaumatin and aspartame. The flavouring and/or sweetening agents may be used alone or in combination with each other.

In a second aspect, the present invention provides a solid-dosage form pharmaceutical composition for oral administration as hereinbefore described for use in the treatment of conditions associated with cephalic pain such as cluster headache, chronic paroxysmal hemicrania, headache associated with vascular disorders, headache associated with substances or their withdrawal, rebound headache, tension headache and, in particular, migraine. Suitably, the 5HT₁ receptor agonist or a pharmaceutically acceptable derivative thereof is sumatriptan or naratriptan or a pharmaceutically acceptable derivative thereof, preferably sumatriptan or a pharmaceutically acceptable derivative thereof, more preferably sumatriptan succinate (1:1) salt.

It will be appreciated that reference to treatment is intended to include prophylaxis as well as the alleviation of established symptoms.

According to a third aspect of the present invention, there is provided the use of a solid-dosage form pharmaceutical composition for oral administration as hereinbefore described in the manufacture of a medicament for treating conditions associated with cephalic pain such as cluster headache, chronic paroxysmal hemicrania, headache associated with vascular disorders, headache associated with substances or their withdrawal, rebound headache, tension headache and, in particular, migraine. Suitably, the 5HT₁ receptor agonist or a pharmaceutically acceptable derivative thereof is sumatriptan or naratriptan or a pharmaceutically acceptable derivative thereof, preferably sumatriptan or a pharmaceutically acceptable derivative thereof, more preferably sumatriptan succinate (1:1) salt.

A fourth aspect of the present invention provides a method of treating a mammal, including a human, suffering from or susceptible to conditions associated with cephalic pain such as cluster headache, chronic paroxysmal hemicrania, headache associated with vascular disorders, headache associated with substances or their withdrawal, rebound headache, tension headache and, in particular, migraine, which comprises oral administration of a solid-dosage form pharmaceutical composition as hereinbefore described. Suitably, the 5HT₁ receptor agonist or a pharmaceutically acceptable derivative thereof is sumatriptan or naratriptan or a pharmaceutically acceptable derivative thereof, preferably sumatriptan or a pharmaceutically acceptable derivative thereof, more preferably sumatriptan succinate (1:1) salt.

It will be appreciated that the amount of compounds employed as the active ingredient in the solid-dosage form compositions of the invention will depend on the particular compounds used. Furthermore, the precise therapeutic dose employed will depend on the age and condition of the patient and the nature of the condition to be treated and will be at the ultimate discretion of the attendant physician.

When the 5HT₁ receptor agonist is sumatriptan or naratriptan, the amount of compound employed in the compositions of the invention will be in the range of 0.1 mg to 250 mg. The compositions may be administered for example 1 to 4 times per day, preferably once or twice. When the 5HT₁ receptor agonist is sumatriptan, the amount of sumatriptan, preferably in the form of a pharmaceutically acceptable salt, will be in the range of 1 mg to 200 mg, preferably 20 mg to 150 mg, for example 25, 50 or 100 mg, expressed as the weight of free base. When the 5HT₁ receptor agonist is naratriptan, the amount of naratriptan, preferably in the form of a pharmaceutically acceptable salt, will be in the range of 0.1 mg to 25 mg, preferably 1 or 2.5 mg, expressed as the weight of free base. Typically in the treatment of cephalic pain, in particular migraine, the composition is administered as a single dose; if a patient experiences rebound pain or recurrance then a subsequent single dose may be administered after a suitable period in accordance with the instructions provided by the attendant physician. Thus, the treatment of cephalic pain, in particular migraine, envisaged in the context of the present invention is essentially by means of acute, single dose, administration of the active ingredient.

Thus, for the preparation of compositions according to the invention, the active ingredient, the acid component, if any, and the base component may be blended with suitable excipients such as insoluble filler and disintegrant. If desired, one or more of the components of this powder blend may be granulated. Tablets may be prepared, for example, by compression of the powder blend using, if required, a lubricant such as magnesium stearate as an aid to tabletting. Capsules may be prepared, for example, by filling of the powder blend into suitable fast-dissolving capsule shells using, if required, a lubricant such as magnesium stearate as an aid to capsule filling.

The solid-dosage form (particularly a tablet) may then be film-coated using a suspension comprising a suitable polymer in a suitable solvent. The preferred solvent for the film coating components is purified water but various classes of organic solvents commonly used in this art such as alcohols, ketones, ethers and chlorinated hydrocarbons, for example ethanol, acetone, dichloromethane and the like, may also be used. The solvent does not appear in the final product.

It is highly desirable in the treatment of acute conditions such as migraine that pharmaceutical compositions have good bioavailability and a rapid onset of action. The solid-dosage form compositions of the present invention have been determined to have excellent disintegration and dispersion properties which result in improved pharmacokinetic parameters. When compared to conventional tablet formulations, the compositions of the present invention disintegrate and disperse more rapidly, resulting in the active ingredient being more rapidly released. This increased speed of drug release leads to faster absorption into the plasma and is thus expected to lead to an enhanced onset of action.

To illustrate the invention further, Tables 1A and 1 B show dissolution data for compositions of the present invention compared with a conventional tablet composition. All compositions contained sumatriptan succinate (1:1) salt as the active ingredient and were film-coated.

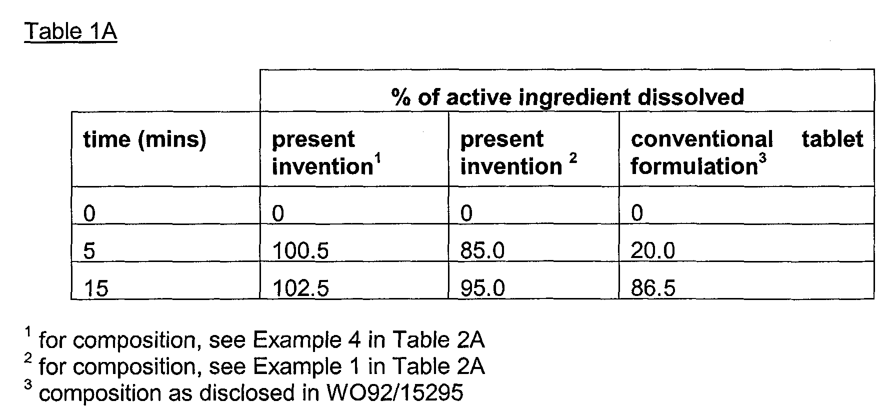

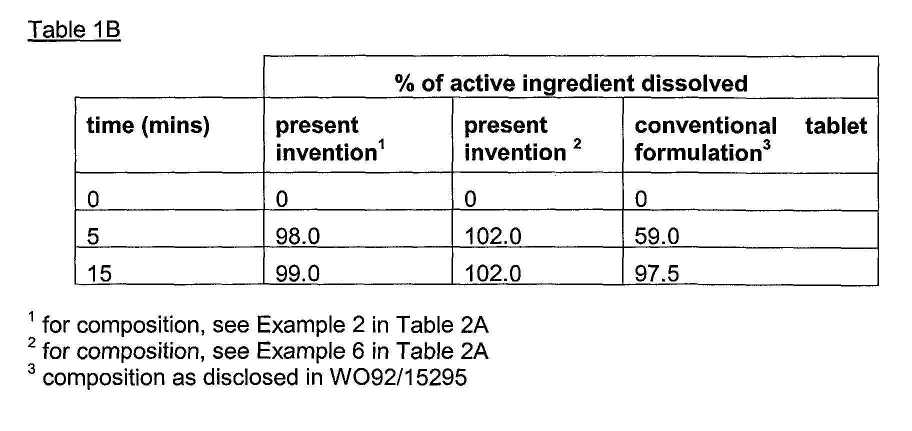

The data in Table 1A were generated on USP II apparatus, according to USP (25-NF20 Supplement 2) <711 Dissolution>, at the discriminating paddle speed of 10 rpm, using 900 mL of simulated gastric fluid (SGF) as dissolution medium at 37.0 °C ± 0.5 °C. SGF comprised 0.01 M aqueous hydrochloric acid with 2 g/L sodium chloride. Such dissolution tests are typically carried out at 50 rpm but the discriminating paddle speed of 10 rpm was chosen as a model of a patient with reduced gastric motility. A similar dissolution profile was shown when water was used as the dissolution medium.

The data in Table 1B were generated on USP II apparatus, according to USP (25-NF20 Supplement 2) <711 Dissolution>, at the discriminating paddle speed of 30 rpm using the same dissolution medium.

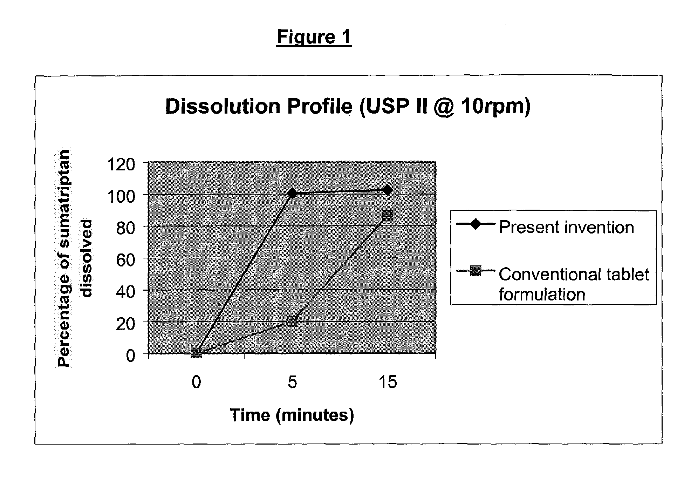

Figure 1 illustrates graphically the dissolution profile generated on USP II apparatus at the discriminating paddle speed of 10rpm of the formulation of the present invention (Example 4) as compared to the corresponding formulation disclosed in WO92/15295 (i.e. as shown in Table 1A).

Thus, in a fifth aspect of the present invention, there is provided the use of a base component of an effervescent couple comprising from about 5 to about 50 % by weight, preferably about 7 to about 20 %, more preferably about 8 to about 15 %, most preferably about 9 to about 12 %, a disintegrant comprising from about 0.5 to about 10 % by weight, preferably about 2 to about 8 %, more preferably about 3 to about 7 %, most preferably about 4 to about 6 %, particularly about 5 %, an insoluble filler comprising from about 30 to 99 % by weight, preferably from about 35 to 80 %, more preferably from about 40 to 70 %, especially about 45 to about 65 %, which includes a wicking agent which itself comprises from about 1 to about 99 % by weight, preferably about 1 to about 80 %, more preferably about 5 to about 65 %, especially about 12 to 55 %, more especially about 18 to 50 %, based on the dry weight of the dosage form, in the preparation of a solid-dosage form pharmaceutical composition for oral administration of a 5HT₁ receptor agonist or a pharmaceutically acceptable derivative thereof, which composition is capable of rapid disintegration and dispersion in the gastrointestinal tract, and/or which composition is capable of providing rapid *in vivo* absorption of the active ingredient upon single dose administration.

Accordingly, as a sixth aspect of the present invention there is provided a solid-dosage form pharmaceutical composition for oral administration comprising a compound which acts as a 5HT₁ receptor agonist or a pharmaceutically acceptable derivative thereof as active ingredient wherein greater than about 70 %, preferably greater than about 80%, more preferably greater than about 90%, of the active ingredient is dissolved in simulated gastric fluid (SGF) within five minutes in USPII apparatus at the discriminating paddle speed of about 10 rpm.

Accordingly, as a seventh aspect of the present invention there is provided a solid-dosage form pharmaceutical composition for oral administration comprising a compound which acts as a 5HT₁ receptor agonist or a pharmaceutically acceptable derivative thereof as active ingredient wherein greater than about 90 %, preferably greater than about 95%, of the active ingredient is dissolved in simulated gastric fluid (SGF) within five minutes in USPII apparatus at the discriminating paddle speed of about 30 rpm.

The non-limiting examples in Tables 2A and 2B illustrate compositions according to the invention in which the 5HT₁ receptor agonist is sumatriptan, present as its succinate (1:1) salt. Other compounds which act as the 5HT₁ receptor agonist may be formulated in a similar manner. Thus, sumatriptan succinate may be replaced by any of the suitable 5HT₁ receptor agonists described herein, such as, for example, naratriptan, zolmitriptan, eletriptan, rizatriptan, frovatriptan, almotriptan, avitriptan, donitriptan, alniditan, ALX-0646, LY334370, U1092291, IS159 and PNY142633.

Furthermore, these non-limiting examples illustrate compositions according to the invention wherein dibasic calcium phosphate and/or microcrystalline cellulose are employed as insoluble fillers with the microcrystalline cellulose acting as the wicking agent, croscarmellose sodium is employed as the disintegrant, sodium bicarbonate is the base component of the effervescent couple, sumatriptan succinate and/or citric acid is the acid component of the effervescent couple, and magnesium stearate is a lubricant.

The data provided in Tables 1, 2A and 2B furthermore illustrate how specific quantities of base component of an effervescent couple, disintegrant and insoluble filler may be combined with a 5HT₁ receptor agonist as active ingredient to prepare a pharmaceutical composition with rapid disintegration and dispersion properties.

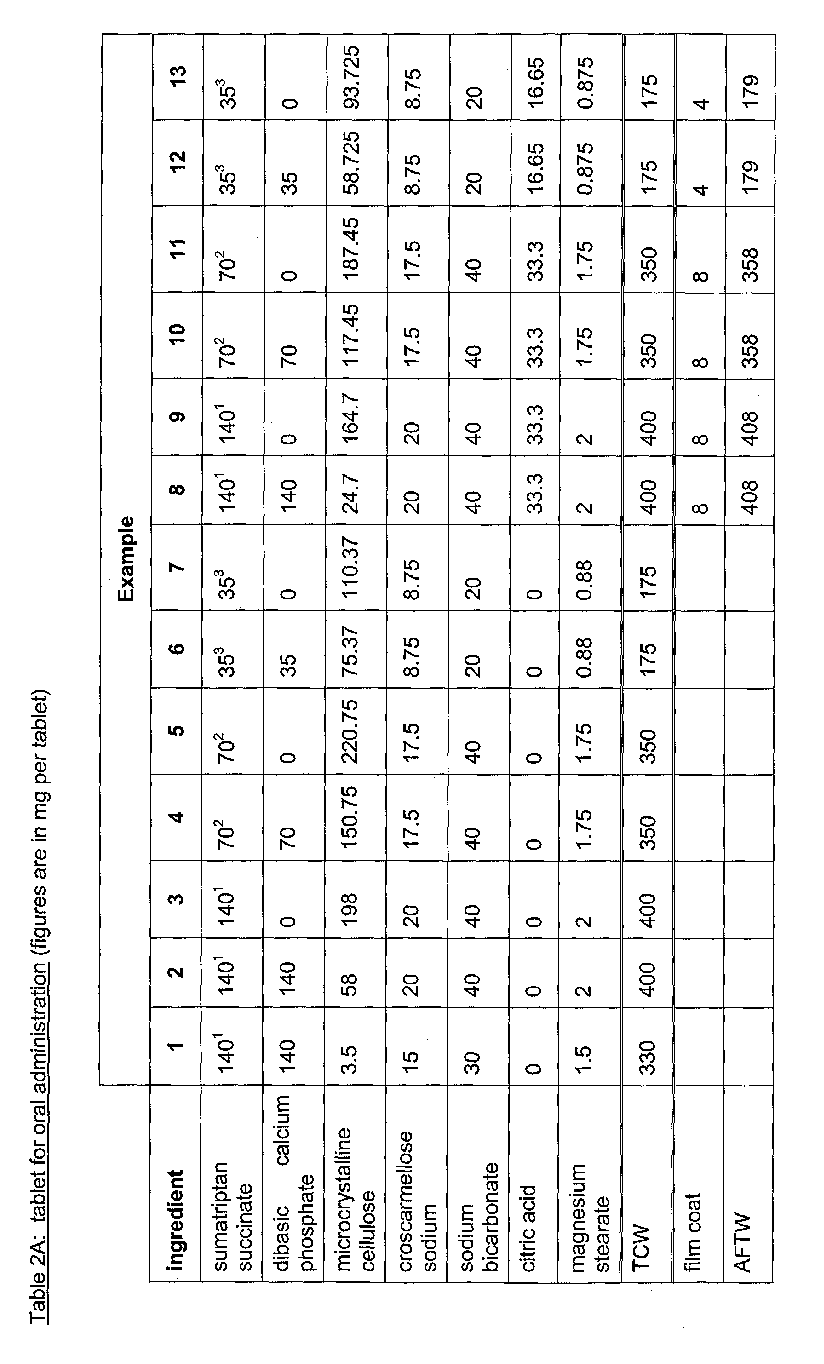

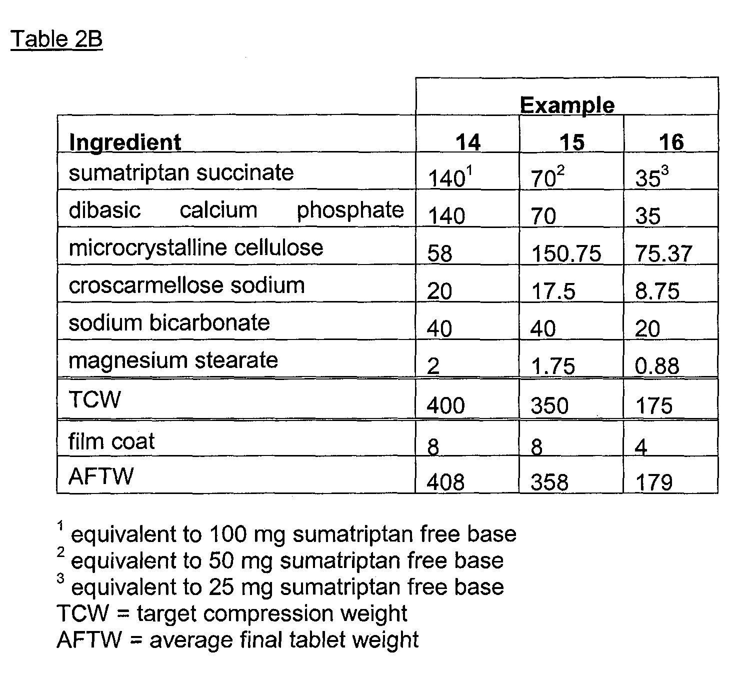

To illustrate the invention further, pharmacokinetic data has been obtained comparing (by means of relevant human trials) solid-dosage formulations of the present invention with corresponding conventional tablet formulations.

The particular pharmacokinetic characteristics referred to hereinafter will be calculated as either mean values or median values, as appropriate (but preferably as indicated below in Tables 3 to 6) for each characteristic, from the individual values obtained for each person (whether a human volunteer or a human patient) studied. In general, such mean or median values will be calculated using trials containing at least 15 people, preferably at least 20 people, and typically between 15 and 35 people, more typically between 20 and 35 people.

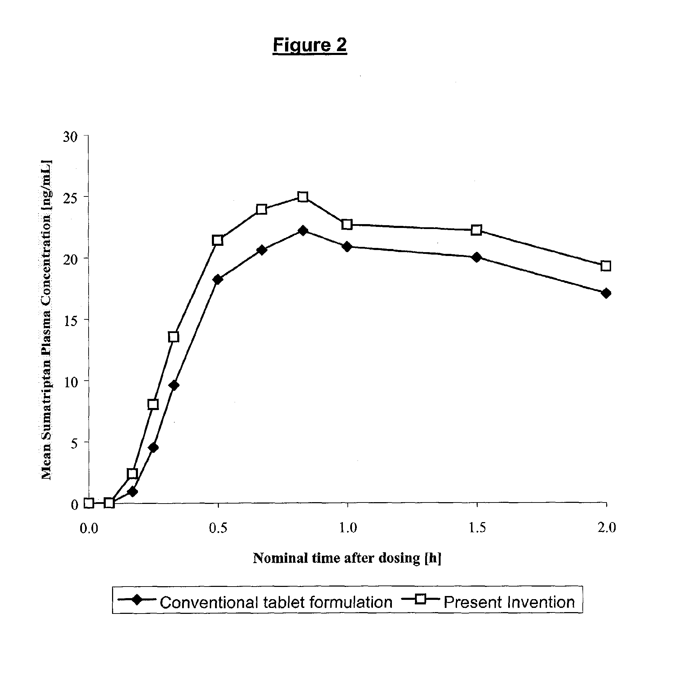

Figure 2 shows the mean plasma concentration-time profiles *in vivo* for sumatriptan following a study in 24 healthy volunteers in a fasted state of single oral administration of a standard tablet of sumatriptan or a tablet formulated according to the present invention, both tablets containing 50 mg of sumatriptan (calculated as sumatriptan base); both tablets contained sumatriptan 1:1 succinate as active ingredient, and both tablets were film-coated.

Table 3 shows the mean plasma concentration-time profiles from the study illustrated by Figure 2 for the first 30 minutes post-dose.

**Table 3**

| **Time (minutes)** | **Mean plasma concentration (ng/mL)** | |
|---|---|---|
| | **Present invention**^{**1**} | **Conventional formulation** ^{**2**} |
| 5 | 0.18 | 0.06 |
| 10 | 2.40 | 0.93 |
| 15 | 8.03 | 4.53 |
| 20 | 13.53 | 9.61 |
| 30 | 21.41 | 18.19 |

| | | |
|---|---|---|
| ¹ for composition, see Example 15 in Table 2B | | |
| ² composition as disclosed in WO92/15295 | | |

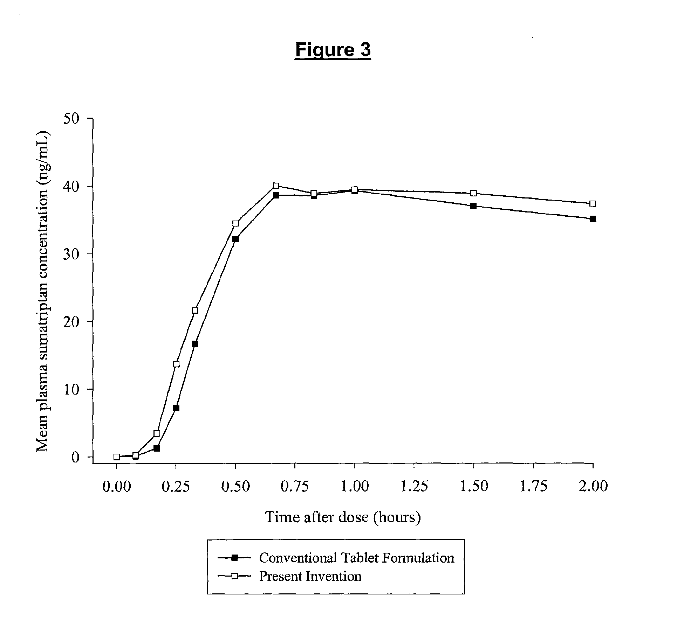

Figure 3 shows the mean plasma concentration-time profiles *in vivo* for sumatriptan following a study in 31 healthy volunteers in a fasted state of single oral administration of a standard tablet of sumatriptan or a tablet formulated according to the present invention, both tablets containing 100 mg of sumatriptan (calculated as sumatriptan base); both tablets contained sumatriptan 1:1 succinate as active ingredient, and both tablets were film-coated.

Table 4 shows the mean plasma concentration-time profiles from the study illustrated by Figure 3 for the first 30 minutes post-dose.

**Table 4**

| **Time (minutes)** | **Mean plasma concentration (ng/mL)** | |
|---|---|---|
| | **Present invention** ^{**1**} | **Conventional formulation** ^{**2**} |
| 5 | 0.24 | 0.10 |
| 10 | 3.45 | 1.25 |
| 15 | 13.65 | 7.17 |
| 20 | 21.60 | 16.66 |
| 30 | 34.45 | 32.11 |

| | | |
|---|---|---|
| ¹ for composition, see Example 14 in Table 2B | | |
| ² composition as disclosed in WO92/15295 | | |

The data from the studies from which Tables 3 and 4 are derived shows that, although the tablet formulations according to the present invention have a similar bioavailability profile to the corresponding conventional tablets based on AUC(0-infinity) and Cmax, the tablet formulations according to the present invention show a marked increase in plasma concentrations achieved during the first 30 minutes after dosing relative to the conventional tablet.

In particular, the tablet formulations of the present invention show significant improvements in a number of parameters characterising this early time period following dosing as compared to the corresponding conventional tablets, as set out below in Table 5 (relating to the study illustrated in Figure 2) and Table 6 (relating to the study illustrated in Figure 3).

**Table 5**

| **Parameter [units]** | **Present invention** ^{**1**} | **Conventional Formulation** ^{**2**} |
|---|---|---|
| AUC (0-0.5h) [ng.h/mL] * | 4.03 | 2.90 |
| T5* [min] | 13 | 16 |
| T10* [min] | 17 | 22 |
| %Cmax, 15 min ** [%] | 27 | 17 |
| %Cmax, 20 min ** [%] | 47 | 36 |
| %Cmax, 30 min ** [%] | 74 | 66 |

| | | |
|---|---|---|
| * - median | | |
| ** - mean T5 - time to reach plasma concentration of 5 ng/mL T10 - time to reach plasma concentration of 10 ng/mL | | |
| ¹ for composition, see Example 15 in Table 2B | | |
| ² corresponding 50mg tablet as disclosed in WO92/15295 | | |

**Table 6**

| **Parameter [units]** | **Present invention** ^{**1**} | **Conventional Formulation** ^{**2**} |
|---|---|---|
| AUC (0-30min) [ng.h/mL] * | 7.01 | 5.49 |
| T5* [min] | 12 | 15 |
| T10*[min] | 16 | 19 |
| %Cmax, 15 min ** [%] | 14 | 27 |
| %Cmax, 20 min ** [%] | 34 | 44 |
| %Cmax, 30 min ** [%] | 63 | 68 |

| | | |
|---|---|---|
| * - median | | |
| ** - mean T5 - time to reach plasma concentration of 5 ng/mL T10 - time to reach plasma concentration of 10 ng/mL | | |
| ¹ for composition, see Example 14 in Table 2B | | |
| ² corresponding 100mg tablet as disclosed in WO92/15295 | | |

Thus, in an eighth aspect of the present invention, there is provided a solid-dosage form pharmaceutical composition for oral administration comprising a 5HT₁ receptor agonist, preferably sumatriptan, or a pharmaceutically acceptable derivative thereof as active ingredient wherein single dose administration in human patients provides an increase of greater than or equal to about 20%, preferably greater than or equal to about 25%, in mean plasma concentration of said 5HT₁ receptor agonist *in vivo* at about 15 minutes post-dose relative to a standard solid-dosage form formulation of said 5HT₁ receptor agonist.

In a particular embodiment, the 5HT₁ receptor agonist is sumatriptan, present as its 1:1 succinate salt, and the increase in mean plasma concentration provided at 15 minutes post-dose relative to the standard formulation is greater than or equal to 20%.

In a further particular embodiment, the 5HT₁ receptor agonist is sumatriptan, present as its 1:1 succinate salt, and the increase in mean plasma concentration provided at 15 minutes post-dose relative to the standard formulation is greater than or equal to 25%.

In a ninth aspect of the present invention, there is provided a solid-dosage form pharmaceutical composition for oral administration comprising a 5HT₁ receptor agonist, preferably sumatriptan, or a pharmaceutically acceptable derivative thereof as active ingredient wherein single dose administration in human patients provides an increase of greater than or equal to about 20%, preferably greater than or equal to about 25%, in mean plasma concentration of said 5HT₁ receptor agonist *in vivo* at about 20 minutes post-dose relative to a standard solid-dosage form formulation of said 5HT₁ receptor agonist.

In a particular embodiment, the 5HT₁ receptor agonist is sumatriptan, present as its 1:1 succinate salt, and the increase in mean plasma concentration provided at 20 minutes post-dose relative to the standard formulation is greater than or equal to 20%.

In a further particular embodiment, the 5HT₁ receptor agonist is sumatriptan, present as its 1:1 succinate salt, and the increase in mean plasma concentration provided at 20 minutes post-dose relative to the standard formulation is greater than or equal to 25%.

In respect of 5HT₁ receptor agonists which are marketed products, the standard solid-dosage form formulations are those which are currently marketed, whether standard swallow-tablet formulations or 'melt in the mouth'/'oral melt' tablet formulations (for example, those marketed as Maxalt™, Maxalt™ MLT, Zomig™, Zomig™ ZLT, Naramig™, and other marketed formulations of the 5-HT₁ receptor agonists referred to above). In particular, in respect of sumatriptan, the standard solid-dosage form formulations are those marketed as Imigran® and Imitrex® tablets (25 mg, 50 mg and 100 mg), and including those formulations described in WO92/15295.

In a tenth aspect of the present invention, there is provided a solid-dosage form pharmaceutical composition for oral administration comprising sumatriptan or a pharmaceutically acceptable derivative thereof as active ingredient, wherein said composition contains 50 mg of sumatriptan (calculated as sumatriptan base), and wherein single dose administration in human patients provides a mean plasma concentration of sumatriptan *in vivo* of greater than or equal to about 7.0 ng/mL, preferably greater than or equal to about 7.5 ng/mL, more preferably greater than or equal to about 8.0 ng/mL at about 15 minutes post-dose.

In an eleventh aspect of the present invention, there is provided a solid-dosage form pharmaceutical composition for oral administration comprising sumatriptan or a pharmaceutically acceptable derivative thereof as active ingredient, wherein said composition contains 50 mg of sumatriptan (calculated as sumatriptan base), and wherein single dose administration in human patients provides a mean plasma concentration of sumatriptan *in vivo* of greater than or equal to about 13.0 ng/mL, preferably greater than or equal to about 13.5 ng/mL, more preferably greater than or equal to about 14.0 ng/mL, at about 20 minutes post-dose.

In a twelfth aspect of the present invention, there is provided a solid-dosage form pharmaceutical composition for oral administration comprising sumatriptan or a pharmaceutically acceptable derivative thereof as active ingredient, wherein said composition contains 100 mg of sumatriptan (calculated as sumatriptan base), and wherein single dose administration in human patients provides a mean plasma concentration of sumatriptan *in vivo* of greater than or equal to about 10.0 ng/mL, preferably greater than or equal to about 11.0 ng/mL, more preferably greater than or equal to about 12.0 ng/mL, most preferably greater than or equal to about 13.0ng/mL, at about 15 minutes post-dose.

In a thirteenth aspect of the present invention, there is provided a solid-dosage form pharmaceutical composition for oral administration comprising sumatriptan or a pharmaceutically acceptable derivative thereof as active ingredient, wherein said composition contains 100 mg of sumatriptan (calculated as sumatriptan base), and wherein single dose administration in human patients provides a mean plasma concentration of sumatriptan *in vivo* of greater than or equal to about 18.0 ng/mL, preferably greater than or equal to about 19.0 ng/mL, more preferably greater than or equal to about 20.0 ng/mL, most preferably greater than or equal to about 21.0 ng/mL, at about 20 minutes post-dose.

In a fourteenth aspect of the present invention, there is provided a solid-dosage form pharmaceutical composition for oral administration comprising a 5HT₁ receptor agonist, preferably sumatriptan, or a pharmaceutically acceptable derivative thereof as active ingredient wherein single dose administration in human patients provides an increase of greater than or equal to about 20%, preferably greater than or equal to about 25%, more preferably greater than or equal to about 30%, most preferably greater than or equal to about 35%, in median AUC(0-0.5h) *in vivo* relative to a standard solid-dosage form formulation of said 5HT₁ receptor agonist.

In a particular embodiment, the 5HT₁ receptor agonist is sumatriptan, present as its 1:1 succinate salt, and the increase in median AUC(0-0.5h) plasma concentration provided relative to the standard formulation is greater than or equal to 20%.

In a further particular embodiment, the 5HT₁ receptor agonist is sumatriptan, present as its 1:1 succinate salt, and the increase in median plasma AUC(0-0.5h) provided relative to the standard formulation is greater than or equal to 25%.

In a further particular embodiment, the 5HT₁ receptor agonist is sumatriptan, present as its 1:1 succinate salt, and the increase in median plasma AUC(0-0.5h) provided relative to the standard formulation is greater than or equal to 30%.

In a further particular embodiment, the 5HT₁ receptor agonist is sumatriptan, present as its 1:1 succinate salt, and the increase in median plasma AUC(0-0.5h) provided relative to the standard formulation is greater than or equal to 35%.

In a fifteenth aspect of the present invention, there is provided a solid-dosage form pharmaceutical composition for oral administration comprising sumatriptan or a pharmaceutically acceptable derivative thereof as active ingredient, wherein said composition contains 50 mg of sumatriptan (calculated as sumatriptan base), and wherein single dose administration in human patients provides a median AUC(0-0.5h) *in vivo* of greater than or equal to about 3.5 ng.h/mL, preferably greater than or equal to about 4.0 ng.h/mL.

In a sixteenth aspect of the present invention, there is provided a solid-dosage form pharmaceutical composition for oral administration comprising sumatriptan or a pharmaceutically acceptable derivative thereof as active ingredient, wherein said composition contains 100 mg of sumatriptan (calculated as sumatriptan base), and wherein single dose administration in human patients provides a median AUC(0-0.5h) *in vivo* of greater than or equal to about 6.0 ng.h/mL, preferably greater than or equal to about 6.5 ng.h/mL, more preferably greater than or equal to about 7.0 ng.h/mL.

In a seventeenth aspect of the present invention, there is provided a solid-dosage form pharmaceutical composition for oral administration comprising a 5HT₁ receptor agonist, preferably sumatriptan, or a pharmaceutically acceptable derivative thereof as active ingredient wherein single dose administration in human patients provides greater than or equal to about 25% of mean Cmax of said 5HT₁ receptor agonist *in vivo* at about 15 minutes post-dose.

In a particular embodiment, the 5HT₁ receptor agonist is sumatriptan, present as its 1:1 succinate salt, and present in an amount of 50mg (calculated as sumatriptan base), and greater than or equal to 25% of mean Cmax is provided at 15 minutes post-dose.

In a particular embodiment, the 5HT₁ receptor agonist is sumatriptan, present as its 1:1 succinate salt, and present in an amount of 100mg (calculated as sumatriptan base) and greater than or equal to 25% of mean Cmax is provided at 15 minutes post-dose.

In an eighteenth aspect of the present invention, there is provided a solid-dosage form pharmaceutical composition for oral administration comprising a 5HT₁ receptor agonist, preferably sumatriptan, or a pharmaceutically acceptable derivative thereof as active ingredient wherein single dose administration in human patients provides greater than or equal to about 40%, preferably greater than or equal to about 45%, of mean Cmax of said 5HT₁ receptor agonist *in vivo* at about 20 minutes post-dose.

In a particular embodiment, the 5HT₁ receptor agonist is sumatriptan, present as its 1:1 succinate salt, and present in an amount of 50mg (calculated as sumatriptan base), and greater than or equal to 40% of mean Cmax is provided at 20 minutes post-dose.

In a particular embodiment, the 5HT₁ receptor agonist is sumatriptan, present as its 1:1 succinate salt, and present in an amount of 50mg (calculated as sumatriptan base), and greater than or equal to 45% of mean Cmax is provided at 20 minutes post-dose.

In a particular embodiment, the 5HT₁ receptor agonist is sumatriptan, present as its 1:1 succinate salt, and present in an amount of 100mg (calculated as sumatriptan base) and greater than or equal to 40% of mean Cmax is provided at 20 minutes post-dose.

In a nineteenth aspect of the present invention, there is provided a solid-dosage form pharmaceutical composition for oral administration comprising a 5HT₁ receptor agonist, preferably sumatriptan, or a pharmaceutically acceptable derivative thereof as active ingredient wherein single dose administration in human patients provides a mean T5 value (time to reach a plasma concentration of 5 ng/mL) *in vivo* post dose of less than or equal to about 90%, preferably less than or equal to about 85%, of the value provided by a standard solid-dosage form formulation of said 5HT₁ receptor agonist.

In a particular embodiment, the 5HT₁ receptor agonist is sumatriptan, present as its 1:1 succinate salt, and present in an amount of 50 mg (calculated as sumatriptan base) and the mean T5 value post-dose relative to the standard formulation is less than or equal to about 90%.

In a particular embodiment, the 5HT₁ receptor agonist is sumatriptan, present as its 1:1 succinate salt, and present in an amount of 100 mg (calculated as sumatriptan base) and the mean T5 value post-dose relative to the standard formulation is less than or equal to about 90%.

In a particular embodiment, the 5HT₁ receptor agonist is sumatriptan, present as its 1:1 succinate salt, and present in an amount of 50 mg (calculated as sumatriptan base) and the mean T5 value post-dose relative to the standard formulation is less than or equal to about 85%.

In a particular embodiment, the 5HT₁ receptor agonist is sumatriptan, present as its 1:1 succinate salt, and present in an amount of 100 mg (calculated as sumatriptan base) and the mean T5 value post-dose relative to the standard formulation is less than or equal to about 85%.

In a twentieth aspect of the present invention, there is provided a solid-dosage form pharmaceutical composition for oral administration comprising a 5HT₁ receptor agonist, preferably sumatriptan, or a pharmaceutically acceptable derivative thereof as active ingredient wherein single dose administration in human patients provides a mean T10 value (time to reach a plasma concentration of 10 ng/mL) *in vivo* post dose of less than or equal to about 90%, preferably less than or equal to about 85%, more preferably less than or equal to 80%, of the value provided by a standard solid-dosage form formulation of said 5HT₁ receptor agonist.

In a particular embodiment, the 5HT₁ receptor agonist is sumatriptan, present as its 1:1 succinate salt, and present in an amount of 50 mg (calculated as sumatriptan base) and the mean T10 value post-dose relative to the standard formulation is less than or equal to about 90%.

In a particular embodiment, the 5HT₁ receptor agonist is sumatriptan, present as its 1:1 succinate salt, and present in an amount of 100 mg (calculated as sumatriptan base) and the mean T10 value post-dose relative to the standard formulation is less than or equal to about 90%.

In a particular embodiment, the 5HT₁ receptor agonist is sumatriptan, present as its 1:1 succinate salt, and present in an amount of 50 mg (calculated as sumatriptan base) and the mean T10 value post-dose relative to the standard formulation is less than or equal to about 85%.

In a particular embodiment, the 5HT₁ receptor agonist is sumatriptan, present as its 1:1 succinate salt, and present in an amount of 100 mg (calculated as sumatriptan base) and the mean T10 value post-dose relative to the standard formulation is less than or equal to about 85%.

In a particular embodiment, the 5HT₁ receptor agonist is sumatriptan, present as its 1:1 succinate salt, and present in an amount of 50 mg (calculated as sumatriptan base) and the mean T10 value post-dose relative to the standard formulation is less than or equal to about 80%.

In a particular embodiment, the 5HT₁ receptor agonist is sumatriptan, present as its 1:1 succinate salt, and present in an amount of 100 mg (calculated as sumatriptan base) and the mean T10 value post-dose relative to the standard formulation is less than or equal to about 80%.

As indicated above, the formulations of the present invention have been designed to provide faster onset of action and/or higher efficacy rates for patients suffering from cephalic pain (and in particular migraine).

In a twenty-first aspect of the present invention there is provided a solid-dosage form pharmaceutical composition for oral administration comprising a 5HT₁ receptor agonist, preferably sumatriptan, or a pharmaceutically acceptable derivative thereof as active ingredient, wherein single dose administration in human patients suffering from cephalic pain, particularly migraine, provides an onset of action against such pain between about 25 minutes and about 15 minutes, preferably between about 20 minutes and about 15 minutes, more preferably at about 15 minutes, post dose.

In a particular embodiment, the 5HT₁ receptor agonist is sumatriptan, present as its 1:1 succinate salt, and present in an amount of 50 mg (calculated as sumatriptan base) and the time to onset of action is between about 25 minutes and about 20 minutes, preferably at about 20 minutes, more preferably between about 20 minutes and about 15 minutes, most preferably at about 15 minutes, post dose.

In a particular embodiment, the 5HT₁ receptor agonist is sumatriptan, present as its 1:1 succinate salt, and present in an amount of 100 mg (calculated as sumatriptan base) and the time to onset of action is between about 25 minutes and about 20 minutes, preferably at about 20 minutes, more preferably between about 20 minutes and about 15 minutes, most preferably at about 15 minutes, post dose.

In a twenty-second aspect of the present invention there is provided a method of treatment of cephalic pain, particularly migraine, in a human patient, comprising the oral administration, as a single dose, of a solid-dosage form pharmaceutical composition as described hereinbefore, said composition comprising a 5HT₁ receptor agonist, preferably sumatriptan, or a pharmaceutically acceptable derivative thereof as active ingredient, and wherein said method results in an onset of action against such pain between about 25 minutes and about 15 minutes, preferably between about 20 minutes and about 15 minutes, more preferably at about 15 minutes, post dose.

In a typical clinical trial studying the efficacy of acute treatment for cephalic pain (especially migraine), the pain is assessed using a standard 4-point categorical scale (IHS Guidelines for Clinical Trials, Cephalagia, 20, 2000, pp765-786); the scale thus includes severe pain, moderate pain, mild pain and no pain.

Onset of action is indicated either by assessment (i) which is the time for patients to go one-point downwards on the 4-point pain scale e.g. severe to moderate, or by assessment (ii) (the more traditional measure of headache pain relief) which is the time for patients to go from severe pain or moderate pain to mild pain or no pain. Assessment (ii) (also referred to in the IHS Guidelines referenced above) can be seen to be a more challenging requirement; as such, the time taken to achieve onset of action by assessment (ii) may well be greater in any particular clinical trial than the time taken to achieve onset of action by assessment (i). When assessment (ii) is used, the compositions and methods of the present invention will typically achieve an onset of action between about 25 minutes and about 20 minutes, preferably at about 20 minutes post dose, whereas when assessment (i) is used, the compositions and methods of the present invention will typically achieve an onset of action between about 25 minutes and about 15 minutes, preferably between about 20 minutes and about 15 minutes, more preferably at about 15 minutes, post dose.

Onset of action is determined when a statistically significant difference from placebo in pain relief is seen. The proportion of patients who obtain pain relief on the active ingredient under investigation and on placebo are typically compared using the Mantel-Haenszel method to determine when the necessary statistical significance has been shown.

The actual measurement of pain-relief efficacy in such clinical trials is typically carried out in two ways; the first way uses a stopwatch, whereby a patient indicates the exact time they notice an improvement (e.g. when their pain goes from "severe" to "moderate"), whereas the second way involves a health professional soliciting the patient's pain level at discrete time points (e.g., 15 min, 20 min, 25 min). The IHS Guidelines referenced above discusses both of these measurement methods.

In a twenty-third aspect of the present invention there is provided a solid-dosage form pharmaceutical composition for oral administration comprising a 5HT₁ receptor agonist, preferably sumatriptan, or a pharmaceutically acceptable derivative thereof as active ingredient, wherein single dose administration in human patients suffering from cephalic pain, particularly migraine, provides headache relief at 2 hours post-dose in greater than or equal to 55%, preferably greater than equal to 60%, more preferably greater than or equal to 65%, most preferably greater than or equal to 70%, and especially preferably greater than or equal to 75%, of patients.

In a twenty-fourth aspect of the present invention there is provided a method of treatment of cephalic pain, particularly migraine, in a human patient, comprising the oral administration, as a single dose, of a solid-dosage form pharmaceutical composition as described hereinbefore, said composition comprising a 5HT₁ receptor agonist, preferably sumatriptan, or a pharmaceutically acceptable derivative thereof as active ingredient, and wherein said method provides headache relief at 2 hours post-dose in greater than or equal to 55%, preferably greater than equal to 60%, more preferably greater than or equal to 65%, most preferably greater than or equal to 70%, and especially preferably greater than or equal to 75%, of patients.

In a particular embodiment, the 5HT₁ receptor agonist is sumatriptan, present as its 1:1 succinate salt, and present in an amount of 50 mg (calculated as sumatriptan base) and headache relief at 2 hours post-dose is provided in greater than or equal to 55%, preferably greater than or equal to 60%, more preferably greater than or equal to 65%, most preferably greater than or equal to 70% of patients.

In a particular embodiment, the 5HT₁ receptor agonist is sumatriptan, present as its 1:1 succinate salt, and present in an amount of 100 mg (calculated as sumatriptan base) and headache relief at 2 hours post-dose is provided in greater than or equal to 55%, preferably greater than equal to 60%, more preferably greater than or equal to 65%, most preferably greater than or equal to 70%, and especially preferably greater than or equal to 75%, of patients.

In a preferred embodiment according to any one of the sixth to twenty-fourth aspects of the present invention, the solid-dosage form pharmaceutical compositions include a base component of an effervescent couple in an amount of about 5 to about 50% by weight, based on the dry weight of the composition.

In a more preferred embodiment according to any one of the sixth to twenty-fourth aspects of the present invention, the solid-dosage form pharmaceutical compositions are those described hereinbefore including the base component of an effervescent couple, a disintegrant and an insoluble filler.

In a most preferred embodiment according to any one of the sixth to twenty-fourth aspects of the present invention the 5HTᵢ receptor agonist is sumatriptan or a pharmaceutically acceptable derivative thereof, especially sumatriptan (1:1) succinate.

It can be seen that the times for onset of action post-dose and/or the % of patients in whom headache relief is provided at 2 hours post-dose quoted above for the compositions and methods of the present invention are improved as compared to standard (currently marketed) solid-dosage formulations, and approach and/or reach those values provided (and referred to above) in using injectable and nasal-spray formulations of sumatriptan. Such improvements represent a significant clinical benefit.

The improvements in both pharmacokinetic characteristics and/or clinical characteristics referred to above for the compositions of the present invention are particularly shown in respect of patients who have gastric stasis.

The improvements in both pharmacokinetic characteristics and/or clinical characteristics referred to above for the compositions of the present invention may also be shown in respect of patients in a fed state e.g. who experience a migraine attack within ~2 hours of a meal.

## Claims

1. A solid-dosage form pharmaceutical composition for oral administration comprising sumatriptan or a pharmaceutically acceptable salt or solvate thereof as active ingredient wherein single dose administration in human patients provides an increase of greater than or equal to about 20% in mean plasma concentration of sumatriptan *in vivo* either at about 15 minutes post-dose or at about 20 minutes post-dose relative to a standard solid-dosage form formulation of said active ingredient.

2. A solid-dosage form pharmaceutical composition for oral administration comprising sumatriptan or a pharmaceutically acceptable salt or solvate thereof as active ingredient wherein single dose administration in human patients provides an increase of greater than or equal to about 20% in median AUC(0-0.5h) *in vivo* relative to a standard solid-dosage form formulation of said active ingredient.

3. A solid-dosage form pharmaceutical composition for oral administration comprising sumatriptan or a pharmaceutically acceptable salt or solvate thereof as active ingredient wherein single dose administration in human patients provides greater than or equal to about 25% of mean Cmax of said active ingredient *in vivo* at about 15 minutes post-dose.

4. A solid-dosage form pharmaceutical composition for oral administration comprising sumatriptan or a pharmaceutically acceptable salt or solvate thereof as active ingredient wherein single dose administration in human patients provides greater than or equal to about 40% of mean Cmax of said active ingredient *in vivo* at about 20 minutes post-dose.

5. A solid-dosage form pharmaceutical composition for oral administration comprising sumatriptan or a pharmaceutically acceptable salt or solvate thereof as active ingredient, wherein single dose administration in human patients suffering from cephalic pain, particularly migraine, provides an onset of action against such pain between about 25 minutes and about 15 minutes post dose.

6. A solid-dosage form pharmaceutical composition for oral administration comprising sumatriptan or a pharmaceutically acceptable salt or solvate thereof as active ingredient, wherein single dose administration in human patients suffering from cephalic pain, particularly migraine, provides headache relief at 2 hours post-dose in greater than or equal to 60% of patients.

7. A solid-dosage form pharmaceutical composition for oral administration comprising sumatriptan or a pharmaceutically acceptable salt or solvate thereof as active ingredient, wherein said composition contains 50 mg of sumatriptan (calculated as sumatriptan base), and wherein single dose administration in human patients provides a mean plasma concentration of sumatriptan *in vivo* of either greater than or equal to about 7.0 ng/mL at about 15 minutes post-dose or greater than or equal to about 13.0 ng/mL at about 20 minutes post-dose.

8. A solid-dosage form pharmaceutical composition for oral administration comprising sumatriptan or a pharmaceutically acceptable salt or solvate thereof as active ingredient, wherein said composition contains 100 mg of sumatriptan (calculated as sumatriptan base), and wherein single dose administration in human patients provides a mean plasma concentration of sumatriptan *in vivo* of either greater than or equal to about 10.0 ng/mL at about 15 minutes post-dose or greater than or equal to about 18.0 ng/mL at about 20 minutes post-dose.

9. A solid-dosage form pharmaceutical composition for oral administration comprising sumatriptan or a pharmaceutically acceptable salt or solvate thereof as active ingredient, wherein said composition contains 50 mg of sumatriptan (calculated as sumatriptan base), and wherein single dose administration in human patients provides a median AUC(0-0.5h) *in vivo* of greater than or equal to about 3.5 ng.h/mL.

10. A solid-dosage form pharmaceutical composition for oral administration comprising sumatriptan or a pharmaceutically acceptable salt or solvate thereof as active ingredient, wherein said composition contains 100 mg of sumatriptan (calculated as sumatriptan base), and wherein single dose administration in human patients provides a median AUC(0-0.5h) *in vivo* of greater than or equal to about 6.0 ng.h/mL.

11. A composition as claimed in any one of claims 1 to 10 wherein the active ingredient is sumatriptan (1:1) succinate salt.

12. A composition as claimed in any one of claims 1 to 11 in the form of a tablet.

13. A composition as claimed in claim 12 wherein the tablet is film-coated.

14. A solid-dosage form pharmaceutical composition for oral administration as claimed in any one of claims 1 to 13 for use in the treatment of migraine.

15. The use of a solid-dosage form pharmaceutical composition for oral administration as claimed in any one of claims 1 to 13 in the manufacture of a medicament for treating migraine.
